# EUROPÄISCHE PATENTSCHRIFT

(11) **EP 2 277 469 B1**
(45) Veröffentlichungstag und Bekanntmachung des Hinweises auf die Patenterteilung: **19.09.2012**
(21) Anmeldenummer: 10014104.3
(22) Anmeldetag: 17.05.2005
(51) Int. Cl.: A61B 17/92, A61B 17/16, B25D 9/02, B25D 17/08

(54) **Schlagwerkzeug, insbesondere für den chirurgischen Einsatz**
Percussion tool, in particular for surgical use
Outil de percussion, notamment pour l'utilisation chirurgicale

(43) Veröffentlichungstag der Anmeldung: 26.01.2011
(62) Teilanmeldung aus: 05737626.1
(73) Patentinhaber: IMT Integral Medizintechnik AG, 6373 Ennetbürgen (CH); Grünig&Elmiger AG, 6102 Malters (CH)
(72) Erfinder: Grünig, Daniel, 6102 Malters (CH)
(74) Vertreter: Fischer, Britta Ruth

(56) Entgegenhaltungen:
- EP-A- 0 597 547
- EP-A- 0 617 926
- EP-A- 1 270 951
- WO-A-88/02246
- DE-A1- 3 536 132
- DE-A1- 10 233 694
- DE-C- 168 700
- DE-U- 8 911 061
- DE-U- 9 100 115
- GB-A- 562 138
- US-A- 1 853 128
- US-A- 5 913 860

## Beschreibung

### Hintergrund

Die Erfindung betrifft ein Schlagwerkzeug, insbesondere für den chirurgischen Einsatz, gemäss Oberbegriff des Anspruchs 1.

### Stand der Technik

Ein Schlagwerkzeug dieser Art ist in EP 617 926 beschrieben. Es besitzt einen Antriebsteil und einen Werkzeughalter. Der Antriebsteil übt auf den Werkzeughalter in einer Achsialrichtung wirkende, oszillatorische Kräfte aus.

Ein solches Schlagwerkzeug kann z.B. zur Betätigung einer Knochenraspel oder eines ähnlichen chirurgischen Werkzeugs verwendet werden. Es kann aber auch zum Eintreiben eines Marknagels oder eines anderen Implantats eingesetzt werden. Der Einfachheit halber werden im vorliegenden Text auch derartige Implantate als "Werkzeuge" bezeichnet.

Die vom Antriebsteil erzeugten Vibrationen führen zu starker mechanischer Beanspruchung des Werkzeughalters, und es ist wichtig, dass dieser so gebaut ist, dass er den auftretenden Kräften dauerhaft gewachsen ist.

Zudem ist es auch von Vorteil, wenn das Gerät so gebaut ist, dass die achsialen Kräfte möglichst geradlinig und torsionsfrei auf das Werkzeug übertragen werden.

Bei der Verwendung des Geräts zum Eintrieben eines Marknagels oder eines ähnlichen Implantats wird oft so vorgegangen, dass zuerst ein Draht oder ein anderes dünnes, stabförmiges Bauteil in den zu operierenden Knochen eingetrieben wird. Sodann wird der Marknagel, welcher eine achsiale Öffnung aufweist, eingebracht, wobei der Draht durch die achsiale Öffnung des Marknagels geführt wird. Der Draht dient in diesem Fall als Führung für den Marknagel. Er tritt auf der proximalen Seite aus dem Marknagel aus und wird um das Einschlagwerkzeug herum geführt. Nach dem Einsetzen des Implantats wird der Draht herausgezogen.

WO-A-88/02246 beschreibt ein Schlagwerkzeug mit einem Werkzeughalter und einen Antriebsglied die mittels eines Zugglieds miteinander Verbunden sind. US-A-1853128 beschreibt ein Schlagwerkzeug mit einen Werkzeughalter zur drehfesten Aufnahme eines Werkzeugs, der vier Erhöhungen aufweist zwischen denen je Platz für die Aufnahme von Schultern das Werkzeugs bleibt.

### Darstellung der Erfindung

Der Erfindung stellt sich die Aufgabe, ein Schlagwerkzeug der oben erwähnten Art bereitzustellen, bei dem die Kraft vom Antriebsteil möglichst effizient an den Werkzeughalter übertragen wird.

Diese Aufgabe wird vom Schlagwerkzeug gemäss Anspruch 1 gelöst. Demgemäss ist das Antriebsteil über eine Kupplung mit dem Werkzeughalter verbunden. Die Kupplung besitzt einen ersten und einen zweiten Teil, wobei entweder der erste Teil am Antriebsteil und der zweite Teil am Werkzeughalter oder der erste Teil am Werkzeughalter und der zweite Teil am Antriebsteil angeordnet ist. Der erste Teil verjüngt sich gegen aussen (d.h. in Achsialrichtung gegen den zweiten Teil hin), während der zweite Teil auf dem ersten aufsitzt und sich gegen aussen (d.h. in Achsialrichtung gegen den ersten Teil hin) aufweitet, so dass unter achsialem Zug der zweite Teil vom ersten Teil gespreizt wird. Zum Erzeugen des achsialen Zugs ist ein Zugglied zum Verbinden der beiden Teile vorgesehen. Diese Anordnung erlaubt einen spielfreien Sitz, der eine gute Kraftübertragung an den Werkzeughalter gewährleistet.

Es kann eine durchgehende Längsöffnung vorgesehen sein, welche sich in Achsialrichtung von der Vorderseite des Schlagwerkzeugs bis zu seiner Rückseite durch den Werkzeughalter und den Antriebsteil erstreckt, sodass sich das Schlagwerkzeug besonders zum Einbringen von Marknägeln und ähnlichen Implantaten eignet. Diese Längsöffnung ist dazu geeignet, ein stabförmiges Bauteil, wie z.B. den Draht zum Führen des Implantats, durch das Schlagwerkzeug hindurchzuführen. Dadurch wird die Handhabung des Drahts vereinfacht, da dieser nicht mehr um das Gerät herum geführt werden muss.

Der Werkzeughalter weist eine Mündungsöffnung und einen an der Mündungsöffnung angeordneten Sitz auf, die zur Aufnahme eines Werkzeugs dienen, sodass ein einfach einsetzbares Schlagwerkzeug bereitgestellt wird. Der Sitz besitzt eine senkrecht zur Achsialrichtung verlaufende Aufnahmefläche, von welcher aus symmetrisch um die Mündungsöffnung'mindestens vier Erhöhungen nach vorne über die Aufnahmefläche hervorstehen. Zwischen den Erhöhungen bleibt Platz für die Aufnahme der Werkzeugschultern. Durch die symmetrische Anordnung von mindestens vier derartigen Erhöhungen bilden sich insgesamt mindestens vier Vertiefungen zwischen den Erhöhungen, die geeignet sind, die Werkzeugschultern aufzunehmen. Somit kann das Werkzeug also in unterschiedlichen Positionen montiert werden.

Der Werkzeughalter kann einen Mittelkörper aus Leichtmetall, einen vorderen Abschlussteil aus Stahl und einem hinteren Abschlussteil aus Stahl aufweisen, um ein Schlagwerkzeug mit ruhigem Lauf bereitzustellen. Dank der Verwendung eines Mittelkörpers aus Leichtmetall kann das Gewicht des Werkzeughalters reduziert werden. Auch wird die Schallerzeugung vermindert. Um die auftretenden hohen Kräfte nach hinten (d.h. zum Antriebsteil hin) und nach vorne (d.h. zum Werkzeug hin) ableiten zu können, sind die beiden Abschlussteile aus Stahl gefertigt, da Leichtmetall den in diesen Bereichen vorhandenen Belastungen nur bedingt gewachsen wäre.

Die Begriffe "vorne" bzw. "Vorderseite" und "hinten" bzw. "Hinterseite" werden im vorliegenden Text und in den Ansprüchen so verstanden, dass "vorne" bzw. "Vorderseite" die Seite des vom Werkzeughalter gehaltenen Werkzeugs und hinten bzw. "Hinterseite" die vom Werkzeug abgewandte Seite der Vorrichtung identifiziert.

### Kurze Beschreibung der Zeichnungen

Weitere Ausgestaltungen, Vorteile und Anwendungen der Erfindung ergeben sich aus den abhängigen Ansprüchen und aus der nun folgenden Beschreibung anhand der Figuren. Dabei zeigen:
Fig. 1 einen Schnitt durch das ganze Schlagwerkzeug entlang seiner Längsachse,
Fig. 2 eine Draufsicht von Werkzeughalter und Antriebsglied,
Fig. 3 einen Schnitt entlang Linie A-A von Fig. 2,
Fig. 4 einen Schnitt entlang Linie B-B von Fig. 2,
Fig. 5 einen Schnitt entlang Linie C-C von Fig. 2,
Fig. 6 einen Schnitt entlang Linie D-D von Fig. 2,
Fig. 7 eine Explosionsdarstellung von Werkzeughalter und Antriebsglied,
Fig. 8 eine Ansicht des vorderen Abschlussteils des Werkzeughalters schräg von vorne,
Fig. 9 einen Schnitt durch den Mittelkörper des Werkzeughalters entlang Linie B-B von Fig. 2,
Fig. 10 eine Ansicht des hinteren Abschlussteils des Werkzeughalters schräg von hinten und
Fig. 11 eine Ansicht des Antriebsglieds schräg von vorne.

### Wege zur Ausführung der Erfindung

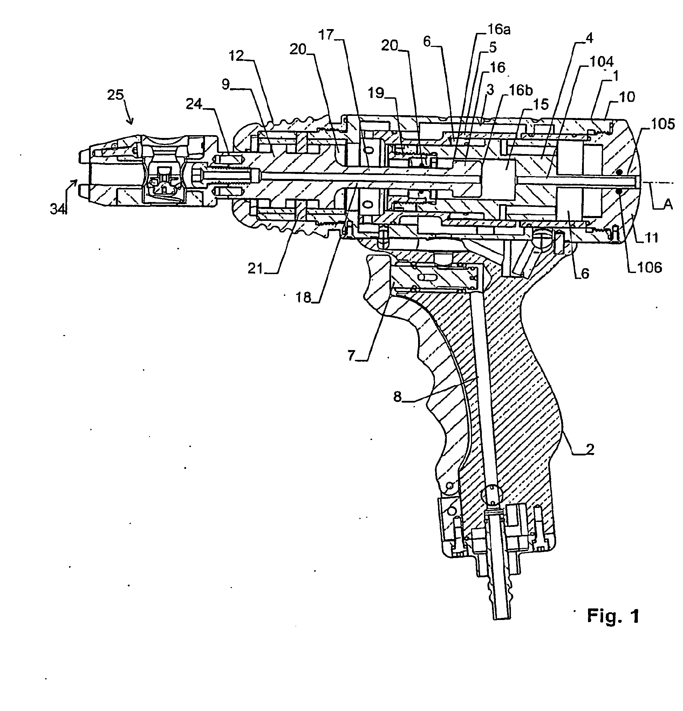

Fig. 1 zeigt eine vorteilhafte Ausführung des erfindungsgemässen Schlagwerkzeugs. Das Werkzeug besitzt ein Gehäuse 1 mit Griff 2. Im Gehäuse 1 ist ein Antriebsteil 3 untergebracht. Dieser umfasst einen Kolben 4, der verschiebbar in einem Zylinder 5 angeordnet ist. Der Kolben 4 befindet sich verschiebbar in einem Zylinderraum 6. Bei Betätigung eines Druckluftventils 7 wird Druckluft über eine Leitung 8 zugeführt, welche den Kolben 4 in oszillierende Bewegung entlang der Längsachse A des Geräts versetzt. Entsprechende Antriebe sind dem Fachmann bekannt. Da die genaue Funktion des Antriebs nicht Gegenstand der vorliegenden Erfindung ist, wird für die Beschreibung eines Ausführungsbeispiels auf EP 617 926 verwiesen. Anstelle eines pneumatischen Antriebs ist auch ein hydraulischer oder ein elektromagnetischer Antrieb denkbar.

Das Gehäuse 1 besitzt eine Wandung 10, welche den Zylinderraum 6 umschliesst und auf die endseitig eine Rückwand 11 und vorne ein Deckel 12 aufgeschraubt sind.

Im Kolben 4 ist ein Hohlraum 15 angeordnet, in welche eine Verdickung 16 eines Antriebsglieds 17 eingreift. Die Verdickung 16 ist mit einer Stange 18 des Antriebsglieds 17 fest verbunden. Die Stange 18 ist durch einen Schraubverschluss 19 mit Dichtung 20 geführt, die in das vordere Ende des Hohlraums 15 ragt.

Die Stange 18 geht an ihrem vorderen Ende in einen Führungsblock 9 des Antriebsglieds 17 über, der in Achsialrichtung A verschiebbar in einer Kammer 20 im vorderen Deckel 12 geführt ist. Die achsiale Bewegung des Antriebsglieds 17 ist durch einen stationären Stopper 21 limitiert, der in eine Nut im Führungsblock 9 eingreift.

Am vorderen Ende ist das Antriebsglied 17 über eine Kupplung 24 mit einem Werkzeughalter 25 verbunden, der dazu dient, ein Werkzeug zu befestigen. Dank der Kupplung 24 kann der Werkzeughalter 25 ohne Auseinandernehmen des Geräts gewechselt werden.

Die Funktionsweise des beschriebenen Geräts entspricht jener gemäss EP 617 926. Durch das oszillatorische Hin- und Herbewegen des Kolbens 4 werden auf die Verdickung 16 oszillatorische Kräfte entlang der Achsrichtung A in Form von Schlägen ausgeübt. Dabei werden abwechslungsweise die Vorderfläche 16a und die Hinterfläche 16b der Verdickung 16 beaufschlagt, so dass das Antriebsglied 17 hin- und herbewegt wird.

Die oszillatorischen Kräfte werden vom Antriebsglied 17 auf den Werkzeughalter 25 übertragen und von dort auf das in den Werkzeughalter eingesetzte Werkzeug.

Im Folgenden werden die einzelne Komponenten des Schlagwerkzeugs im Detail beschrieben.

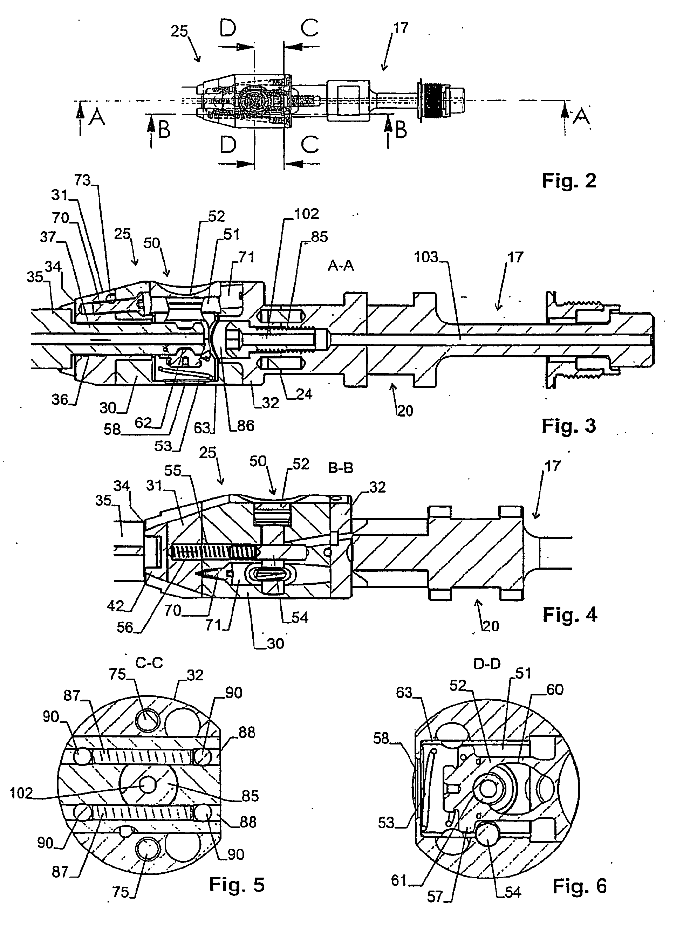

Der Aufbau des Werkzeughalters 25 und des Antriebsglieds 17 ist in Fig. 2 - 7 illustriert.

Der Werkzeughalter 25 umfasst drei Hauptteile, nämlich einen Mittelkörper 30 aus Leichtmetall, einen vorderen Abschlussteil 31 aus Stahl und einen hinteren Abschlussteil 32 ebenfalls aus Stahl. Der Mittelkörper 30 besteht vorwiegend aus Aluminium und/oder Magnesium und/oder Titan, wobei Aluminium aus Preisgründen vorteilhaft ist.

Am vorderen Ende des Werkzeughalters 25 bildet der vordere Abschlussteil 31 einen Sitz 34 zur drehfesten Aufnahme des Werkzeugs 35, wobei vom Werkzeug 35 in Fig. 2 - 7 nur jeweils der hinterste Teil dargestellt ist. Vom Sitz 34 aus erstreckt sich eine Mündungsöffnung 36 in Achsialrichtung in den Werkzeughalter 25 hinein. Diese dient zur Aufnahme eines hinteren, stabförmigen Teils 37 des Werkzeugs 35.

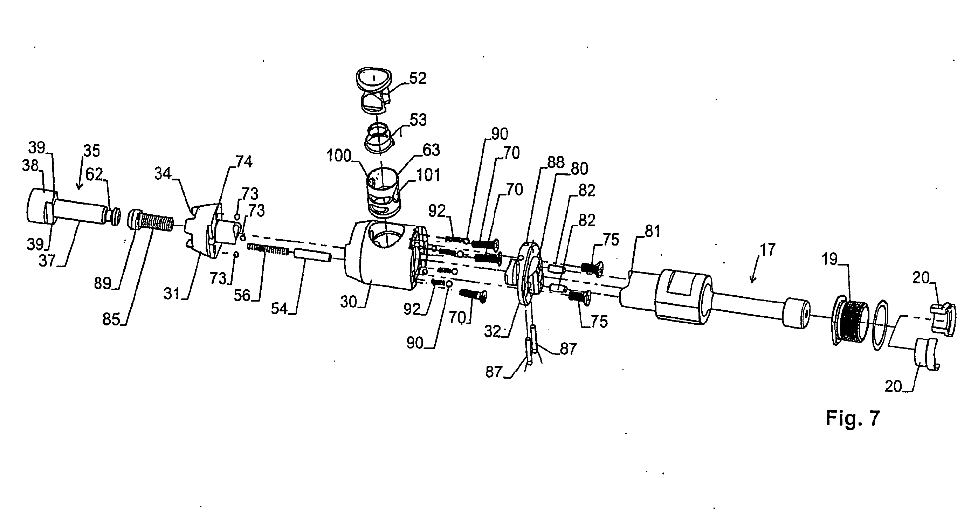

Wie insbesondere aus Fig. 7 gut ersichtlich, geht der stabförmige Teil 37 des Werkzeugs an seinem vorderen Ende in einen breiteren Werkzeugabschnitt 38 über, der zwei seitlich vom stabförmigen Teil 37 abstehende Schultern 39 bildet. Beim eingesetzten Werkzeug 35 werden diesen Schultern drehfest vom Sitz 34 aufgenommen.

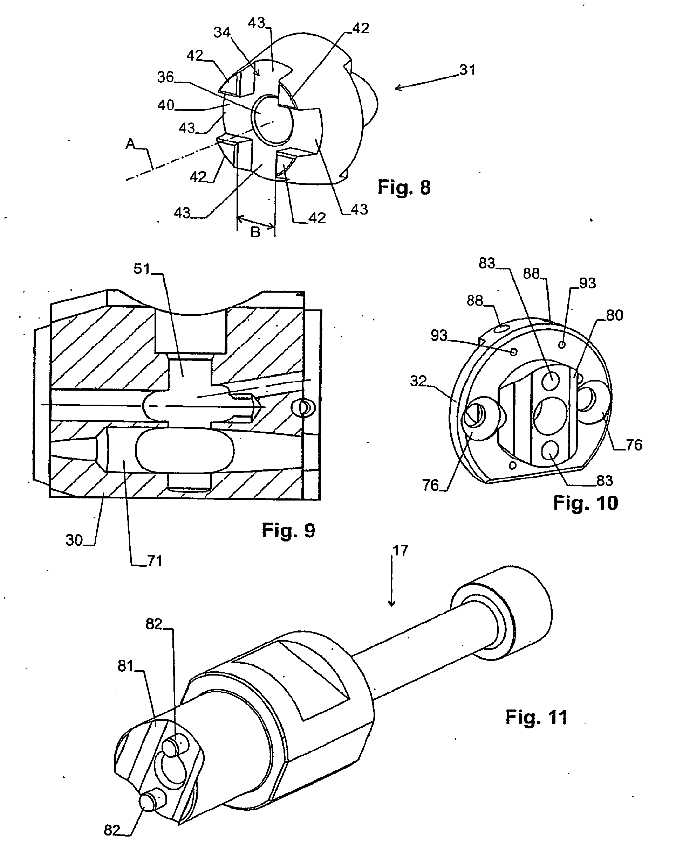

Der hierzu gewählte Aufbau des Sitzes 34 ist aus Fig. 8 besonders klar ersichtlich. Wie dargestellt, weist der Sitz 34 eine senkrecht zur Achsialrichtung A verlaufende Aufnahmefläche 40 auf, mit welcher Stosskräfte vom Werkzeughalter 25 auf die hinteren Abschlussflächen der Schultern 39 übertragen werden können.

Über die Aufnahmefläche 40 stehen mindestens vier, symmetrisch um die Mündungsöffnung 36 angeordnete Erhöhungen 42 in Achsialrichtung A nach vorne vor. Zwischen den Erhöhungen 42 bilden sich auf diese Weise mindestens vier symmetrische Vertiefungen 43, welche die Schultern 39 des Werkzeugs 35 drehfest aufnehmen können.

Bei der in Fig. 8 gezeigten Ausführung mit vier ungefähr dreieckförmigen Erhöhungen 42, bilden die vier Vertiefungen 43 zwei senkrecht zueinander und zur Achsialrichtung A stehende Nuten, die sich im Bereich der Mündungsöffnung 36 schneiden. In diese Nuten kann das Werkzeug 35 in insgesamt vier unterschiedlichen Lagen eingelegt werden.

Vorzugsweise beträgt der Abstand B zwischen benachbarten Erhöhungen 42 ca. 10.05 +/- 0.02 mm. Dies entspricht der Breite der Schultern standardisierter Werkzeuge im chirurgischen Bereich.

Wie insbesondere aus Fig. 3 ersichtlich, erstreckt sich der stabförmigen Teil 37 des Werkzeugs 35 durch die Mündungsöffnung 36 bis in den Mittelkörper 30. Dort wird er von einem Haltemechanismus 50 festgehalten. Hierzu ist der Haltemechanismus 50 mit einem in einer Kammer 51 des Mittelkörpers 30 angeordneten Haltekörper 52 ausgestattet. Der Haltekörper 52 kann in der Kammer 51 quer zur Längsachse A (siehe Fig. 2) gegen die Kraft einer konischen Feder 53 bewegt werden. Hierzu ist die Kammer 51 an einer ersten Seite gegen aussen offen, und die Feder 53 ist an der zweiten Seite der Kammer 51 abgestützt. Der Haltemechanismus 50 bzw. der Haltekörper 52 ist gegen die Kraft der Feder 53 von einen Stift 54 gesichert. Der Stift 54 befindet sich in einem Sackloch 55 des Mittelkörpers 30 und ist in diesem mit einer Feder 56 beaufschlagt. Das Sackloch 55 mündet in die Trennfläche zwischen Mittelkörper 30 und vorderem Abschlussteil 31, so dass der Stift 54 im Sackloch 55 gefangen ist und erst nach dem Trennen von Mittelkörper 30 und vorderem Abschlussteil 31 aus dem Sackloch 55 entnommen werden kann. Wie insbesondere aus Fig. 6 ersichtlich, liegt der Stift 54 gegen eine Nase 57 des Haltekörpers 52 an und hält diesen gegen die Kraft der Feder 53 fest.

Die Kammer 51 weist im Bereich der Feder 53 eine zweite Öffnung 58 auf, wodurch eine Reinigung der Kammer erleichtert wird.

Der Haltekörper 52 besitzt eine zentrale Öffnung 60 (siehe Fig. 6), deren Rand 61 in eine Nut 62 (siehe Fig. 7) im stabförmigen Teil 37 des Werkzeugs 35 eingreift und dieses festhält. Um das Werkzeug zu lösen, muss der Haltekörper 52 einfach gegen die Kraft der Feder 53 in die Kammer 51 gedrückt werden.

Zwischen dem Haltemechanismums 50 bzw. dem Haltekörper 52 und dem Mittelkörper 30 ist in der Kammer 51 ein dünner Stahlmantel 63 angeordnet. Dieser verteilt die vom Haltekörper 52 erzeugten Kräfte auf das Leichtmetall des Mittelkörpers 30 und verhindert, dass dieser beschädigt wird.

Mittelkörper 30 und vorderer Abschlussteil 31 sind über drei Schrauben 70 miteinander befestigt (siehe Fig. 3, 4, 7). Die Schrauben 70 sitzen in schrägen Bohrungen 71 des Mittelkörpers.

Damit sich die Schrauben 70 im Betrieb des Geräts nicht lösen können, sind Fixierkörper 73, z.B. aus Kunststoff, vorgesehen. Die Fixierkörper 73 sitzen in Vertiefungen 74 (Fig. 7) des vorderen Abschlussteils 31, angrenzend an die Löcher für die Gewinde der Schrauben 70. Die Vertiefungen 74 sind so bemessen, dass die Fixierkörper 73 in entspanntem Zustand leicht über die Vertiefungen hinausschauen und beim Anziehen der Schrauben 70 vom Mittelkörper 30 deformiert und dabei gegen die Schrauben 71 gedrückt werden.

Zur Befestigung des hinteren Abschlussteils 32 am Mittelkörper 30 sind zwei Schrauben 75 vorgesehen (Fig. 5, 7). Deren Köpfe liegen in zwei Vertiefungen 76 (Fig. 10) in der Rückseite des hinteren Abschlussteils 32 und sind so positioniert, dass sie vom vorderen Ende des montierten Antriebsglieds 17 teilweise abgedeckt und festgehalten werden, so dass sie im Betrieb sicher fixiert bleiben.

Die Kupplung 24 zwischen dem Werkzeughalter 25 und dem Antriebsglied 17 besitzt, wie eingangs erwähnt, einen ersten und einen zweiten Teil, die ineinander eingreifen. Der erste Teil ist im vorliegenden Beispiel am hinteren Ende des Werkzeughalters 25 angeordnet und wird von einem sich gegen hinten verjüngenden Sattel 80 des hinteren Abschlussteils 32 gebildet (siehe Fig. 7 und 10). Der zweite Teil wird im vorliegenden Beispiel vom vorderen Ende des Antriebsglieds 17 gebildet und hat die Form eines Reiters 81 (Fig. 7 und 11), der auf dem Sattel 80 sitzt und dessen Form angepasst ist. Im Reiter 81 ist zudem zwei Kupplungsstifte 82 gehalten, welche in Löcher 83 (Fig. 10) des hinteren Abschlussteils eingreifen und so eine auch gegen Verschiebung in Sattellängsrichtung formschlüssige Verbindung der beiden Teile bilden.

Somit bildet die Kupplung 24 eine Verbindung, welche bezüglich Drehung um die Längsachse A und Verschiebung senkrecht zur Längsachse A formschlüssig ist.

Zur Befestigung des Werkzeughalters 25 am Antriebsglied 17 ist weiter ein Zugglied in Form einer Kupplungsschraube 85 vorgesehen. Deren Kopf sitzt in einer Vertiefung 86 (Fig. 3) der hinteren Wand der Kammer 51, und ihr Gewindeschaft greift durch die Kupplung 24 in eine achsiale Bohrung des Antriebsglieds 17 ein. Sie zieht somit das Antriebsglied 17 gegen den Werkzeughalter 25. Dabei wird der Reiter 81 auf dem Sattel 80 etwas gespreizt, so dass sich eine stabile, spielfreie Verbindung ergibt.

Die Kupplungsschraube 85 wird von zwei Sicherungskörpern in Form von Sicherungsstiften 87 gesichert (Fig. 5, 7). Diese befinden sich in Stiftlöchern 88 (Fig. 5, 7, 10) des hinteren Abschlussteils 32. Sie durchqueren zwei seitliche Ausnehmungen 89 im Kopf der Kupplungsschraube 85 und verhindern so eine Drehung derselben in formschlüssiger Weise.

Die Sicherungsstifte 87 sind in den Stiftlöchern 88 von kugelförmigen Stiftriegeln 90 gehalten. Jeder der Stiftriegel 90 befindet sich in je einem Riegelloch, welches sich von der Vorderseite des hinteren Abschlussteils 32 her erstreckt. Jedes Riegelloch schneidet eines der Stiftlöcher 88 und verjüngt sich dort, um einen Sitz für einen der Stiftriegel 90 zu bilden. In jedem Riegelloch ist ferner eine Feder 92 (Fig. 7) angeordnet, um den jeweiligen Stiftriegel 90 gegen diesen Sitz zu drücken. Damit der Stiftriegel 90 gelöst werden kann, erstreckt sich jedes Riegelloch mit reduziertem Durchmesser bis zu einer Öffnung 93 (Fig. 10) in der hinteren Endfläche des hinteren Abschlussteils 32, durch welche ein Stössel eingeführt werden kann, um den Stiftriegel 90 gegen die Kraft der Feder 92 nach vorne aus dem Stiftloch 88 zu drücken.

Das Antriebsglied 17 ist einstückig ausgeführt und erstreckt sich vom Werkzeughalter 25 bis in den Antriebsteil 3, so dass eine einwandfreie Kraftübertragung ohne Torsionskomponenten gewährleistet ist.

Wie eingangs bereits erwähnt, ist mit Vorteil eine Längsöffnung vorgesehen, welche sich von der Vorderseite des Schlagwerkzeugs bis zu dessen Rückseite erstreckt. Sie dient dazu, einen Führungsdraht oder dergleichen durch das Werkzeug zu führen.

Die Längsöffnung beginnt an der Mündungsöffnung 36, durchtritt eine vordere Öffnung 100 (Fig. 7) im Stahlmantel 63, die zentrale Öffnung 60 des Haltekörpers 52, eine hintere Öffnung 101 im Stahlmantel 63, eine Längsbohrung 102 (Fig. 3) der Kupplungsschraube 85 und eine Längsbohrung 103 des Antriebsglieds 17 (Fig. 3). Von dort erstreckt sich die Längsöffnung durch den Hohlraum 15 (Fig. 1). Hinter dem Hohlraum 15 ist eine Öffnung 104 im Kolben 4 vorgesehen, welche in ein Rohr 105 mündet, das an der Rückseite des Kolbens 4 angeordnet ist und sich von diesem nach hinten erstreckt. Dieses Rohr ist in Achsrichtung A verschiebbar durch eine Dichtung 106 in der Rückwand 11 geführt.

Um die Funktion des pneumatischen Systems des Antriebsteils 3 nicht zu behindern, muss das Innere der Längsöffnung abgedichtet sein. Nebst der Dichtung 106, welche den Zylinderraum 6 gegen aussen isoliert, dient hierzu die Dichtung 20, welche dazu dient, den Hohlraum 15 und die Längsöffnung gegen den Zylinderraum 6 abzudichten.

Während in der vorliegenden Anmeldung bevorzugte Ausführungen der Erfindung beschrieben sind, ist klar darauf hinzuweisen, dass die Erfindung nicht auf diese beschränkt ist und in auch anderer Weise innerhalb des Umfangs der folgenden Ansprüche ausgeführt werden kann.

## Patentansprüche

1. Schlagwerkzeug, insbesondere für den chirurgischen Einsatz, mit
einem Antriebsteil (3),
einem mit dem Antriebsteil (3) verbundenen Antriebsglied (17) und
einem auf einer Vorderseite des Antriebsteils (3) angeordneten, mit dem Antriebsglied (17) verbundenen Werkzeughalter (25),
wobei der Antriebsteil (3) ausgestaltet ist, um über das Antriebsglied (17) auf den Werkzeughalter (25) in einer Achsialrichtung (A) wirkende, oszillatorische Kräfte auszuüben,
**dadurch gekennzeichnet, dass** das Antriebsglied (17) über eine Kupplung (24) mit dem Werkzeughalter (25) verbunden ist, wobei die Kupplung (24) einen sich gegen aussen verjüngenden ersten Teil (80) und einen auf dem ersten Teil aufsitzenden, sich gegen aussen aufweitenden zweiten Teil (81) aufweist, wobei unter achsialen Zug der zweite Teil auf dem ersten Teil aufsitzt und vom ersten Teil gespreizt wird,
dass zwischen dem Werkzeughalter (25) und dem Antriebsglied (17) ein Zugglied (85) zum Verbinden der Teile und zum Erzeugen des achsialen Zugs angeordnet ist und
dass der Werkzeughalter (25) eine Mündungsöffnung und einen an der Mündungsöffnung (36) angeordneten Sitz zur drehfesten Aufnahme eines Werkzeugs aufweist, wobei der Sitz eine senkrecht zur Achsialrichtung (A) verlaufende Aufnahmefläche (40) aufweist, von welcher symmetrisch um die Mündungsöffnung (36) mindestens vier Erhöhungen (42) nach vorne über die Aufnahmefläche (40) hervorstehen, zwischen denen je Platz für die Aufnahme von Schultern des Werkzeugs bleibt.

2. Schlagwerkzeug nach Anspruch 1, wobei die Kupplung (24) formschlüssig gegen Drehung um die Achsialrichtung (A) ausgestaltet ist und insbesondere wobei der erste Teil (80) eine sattelförmige Erhöhung aufweist.

3. Schlagwerkzeug nach Anspruch 1 oder 2, wobei das Zugglied (85) eine sich durch die Kupplung (24) erstreckende Kupplungsschraube (85) aufweist, und insbesondere wobei sich die Längsöffnung (102) durch die Kupplungsschraube (85) erstreckt.

4. Schlagwerkzeug nach Anspruch 3, wobei die Kupplungsschraube (85) von mindestens einem Sicherungskörper formschlüssig gegen Verschiebung gesichert ist, und insbesondere wobei der Sicherungskörper ein Sicherungsstift (87) ist, welcher in einem Stiftloch (88) im hinteren Abschlussteil (32) sitzt und von mindestens einem Stiftriegel (90) gehalten ist, und insbesondere wobei der Stiftriegel (90) einen in einem Riegelloch (93) im hinteren Abschlussteil (32) angeordneten, gegen eine Federkraft gehaltenen Riegelkörper aufweist, wobei der Riegelkörper gegen die Federkraft aus dem Stiftloch (88) bewegbar ist.

5. Schlagwerkzeug nach einem der Ansprüche 1 bis 4, wobei die Kupplung (24) mindestens einen Kupplungsstift (82) aufweist, welcher sich parallel zur Achsialrichtung (A) vom Antriebsglied (17) in das Abschlussteil (32) erstreckt.

6. Schlagwerkzeug nach einem der vorangehenden Ansprüche, wobei das Antriebsglied (17) sich einstückig von der Kupplung (24) bis in den Antriebsteil (3) erstreckt.

7. Schlagwerkzeug nach einem der vorangehenden Ansprüche, wobei das Schlagwerkzeug eine sich in Achsialrichtung (A) durch den Werkzeughalter (25) und den Antriebsteil (3) erstreckende, durchgehende Längsöffnung von der Vorderseite des Schlagwerkzeugs zu einer Rückseite des Schlagwerkzeugs zum Durchführen eines stabförmigen Bauteils aufweist.

8. Schlagwerkzeug nach Anspruch 7, wobei der Werkzeughalter (25) eine Mündungsöffnung (36) zur Aufnahme eines Werkzeugendes aufweist, wobei die Längsöffnung in die Mündungsöffnung (36) mündet.

9. Schlagwerkzeug nach Anspruch 7 oder 8, wobei der Werkzeughalter (25) über ein Antriebsglied (17) mit dem Antriebsteil (3) verbunden ist, wobei sich die Längsöffnung durch das Antriebsglied (17) erstreckt.

10. Schlagwerkzeug nach einem der Ansprüche 7 bis 9, wobei der Antriebsteil (3) einen zu oszillierender Bewegung angetriebenen Kolben (4) aufweist, wobei sich die Längsöffnung durch den Kolben (4) erstreckt.

11. Schlagwerkzeug nach den Ansprüchen 9 und 10, wobei das Antriebsglied (17) eine Verdickung (16) aufweist, die in einem Hohlraum (15) im Innern des Kolbens (4) angeordnet ist, wobei die Längsöffnung durch den Hohlraum (15) und eine hinter dem Hohlraum verlaufende Öffnung (104) im Kolben (4) verläuft, und insbesondere wobei der Hohlraum (15) gegenüber einem Zylinderraum (6), in welchem sich der Kolben (4) befindet, durch eine Dichtung (20) abgedichtet ist.

12. Schlagwerkzeug nach Anspruch 11, wobei sich vom Kolben (4) ein Rohr (105) gegen hinten erstreckt, wobei die Längsöffnung durch das Rohr (105) verläuft, und insbesondere wobei sich das Rohr (105) verschiebbar durch eine Dichtung (106) in einer Rückwand (11) des Schlagwerkzeugs erstreckt.

13. Schlagwerkzeug nach einem der vorangehenden Ansprüche, wobei benachbarte Erhöhungen (42) einen Abstand von ca. 10.05 mm aufweisen.

14. Schlagwerkzeug nach einem der vorangehenden Ansprüche, wobei der Werkzeughalter (25) einen Mittelkörper (30) aus Leichtmetall, einen vorderen Abschlussteil (31) aus Stahl und einen hinteren Abschlussteil aus Stahl (32) aufweist.

15. Schlagwerkzeug nach Anspruch 14, wobei der Mittelkörper (30) vorwiegend aus Aluminium und/oder Magnesium und/oder Titan besteht.

16. Schlagwerkzeug nach einem der Ansprüche 14 oder 15, wobei der vordere Abschlussteil (31) einen Sitz zur drehfesten Aufnahme eines Werkzeugs aufweist.

17. Schlagwerkzeug nach einem der Ansprüche 14 bis 16, wobei im Werkzeughalter (25), insbesondere im Mittelkörper (30), ein Haltemechanismus (50) zum Festhalten des durch eine Mündungsöffnung (36) in den Mittelkörper (30) eingreifenden Werkzeugs, insbesondere wobei zwischen dem Haltemechanismus (50) und dem Mittelkörper (30) ein Stahlmantel (63) angeordnet ist, und insbesondere wobei der Haltemechanismus (50) einen Haltekörper (52) aufweist, welcher quer zur Längsachse gegen eine Federkraft verschiebbar ist.

18. Schlagwerkzeug nach Anspruch 17, wobei der Haltemechanismus im Mittelkörper (30) angeordnet ist, und/oder wobei der Haltemechanismus (50) gegen die Federkraft von einem Stift (54) gesichert ist, welcher in einem Sackloch (55) im Mittelkörper (30) angeordnet ist, wobei das Sackloch (55) in eine Trennfläche zwischen dem Mittelkörper (30) und vorderem Abschlussteil (31) mündet, derart, dass der Stift (54) bei montiertem vorderen Abschlussteil (31) im Sackloch (55) gefangen ist.

## Claims

1. Percussive tool, in particular for surgical use, with
a drive part (3),
a drive member (17) that is connected to the drive part (3) and
a tool holder (25) that is arranged on a front side of the drive part (3) and that is connected to the drive member (17),
wherein the drive part (3) is designed to exert oscillatory forces acting in an axial direction (A) onto the tool holder (25) by way of the drive member (17),
**characterized in that** the drive member (17) is connected to the tool holder (25) by way of a coupling (24), wherein the coupling (24) has an outwardly tapering first part (80) and an outwardly extending second part (81) that sits on the first part, wherein the second part sits on the first part under axial tension and is spread apart by the first part,
that a tension member (85) is arranged between the tool holder (25) and the drive member (17) to connect the parts and to generate the axial tension and
that the tool holder (25) has an orifice opening and a seat arranged at the orifice opening (36) for receiving a tool in a rotationally rigid manner, wherein the seat has a receiving face (40) that runs perpendicularly to the axial direction (A), from which at least four elevations (42) project symmetrically around the orifice opening (36) frontally over the receiving face (40), between each of which remains space for receiving shoulders of the tool.

2. Percussive tool according to claim 1, wherein the coupling (24) is designed to be form-locking against rotation around the axial direction (A), and in particular wherein the first part (80) has a saddle-shaped elevation.

3. Percussive tool according to claim 1 or 2, wherein the tension member (85) has a coupling screw (85) extending through the coupling (24), and in particular wherein the longitudinal opening (102) extends through the coupling screw (85).

4. Percussive tool according to claim 3, wherein the coupling screw (85) is retained by at least one retaining body form-lockingly against displacement, and in particular wherein the retaining body is a retaining pin (87) that is seated in a pin hole (88) in the rear terminal part (32) and is held by at least one pin lock bar (90), and in particular wherein the pin lock bar (90) has a lock bar body that is arranged in a lock bar hole (93) in the rear terminal part (32) and that is held against a spring force, wherein the lock bar body is movable out of the pin hole (88) against the spring force.

5. Percussive tool according to one of the claims 1 to 4, wherein the coupling (24) has at least one coupling pin (82), which extends parallel to the axial direction (A) from the drive member (17) into the terminal part (32).

6. Percussive tool according to one of the preceding claims, wherein the drive member (17) extends in one piece from the coupling (24) into the drive part (3).

7. Percussive tool according to one of the preceding claims, wherein the percussive tool has a continuous longitudinal opening extending in the axial direction (A) through the tool holder (25) and the drive part (3) from the front side of the percussive tool to the back side of the percussive tool for passage of a rod-shaped element.

8. Percussive tool according to claim 7, wherein the tool holder (25) has an orifice opening (36) for receiving a tool end, wherein the longitudinal opening opens into the orifice opening (36).

9. Percussive tool according to claim 7 or 8, wherein the tool holder (25) is connected to the drive part (3) by way of a drive member (17), wherein the longitudinal opening extends through the drive member (17).

10. Percussive tool according to one of the claims 7 to 9, wherein the drive part (3) has a piston (4) that is driven to undergo oscillatory movement, wherein the longitudinal opening extends through the piston (4).

11. Percussive tool according to the claims 9 and 10, wherein the tool member (17) has an enlargement (16) that is arranged in a cavity (15) in the interior of the piston (4), wherein the longitudinal opening passes through the cavity (15) and an opening (104) behind the cavity runs in the piston (4), and in particular wherein the cavity (15) is sealed from a cylinder chamber (6), in which the piston (4) is situated, by a seal (20).

12. Percussive tool according to claim 11,
wherein a tube (105) extends rearwards from the piston (4), wherein the longitudinal opening passes through the tube (105), and in particular wherein the tube (105) extends movably through a seal (106) in a rear wall (11) of the percussive tool.

13. Percussive tool according to one of the preceding claims, wherein neighbouring elevations (42) have a distance of approximately 10.05 mm.

14. Percussive tool according to one of the preceding claims, wherein the tool holder (25) has a central body (30) made of light metal, a front terminal part (31) made of steel and a rear terminal part (32) made of steel.

15. Percussive tool according to claim 14,
wherein the central body (30) primarily consists of aluminium and/or magnesium and/or titanium.

16. Percussive tool according to one of the claims 14 or 15, wherein the front terminal part (31) has a seat for receiving a tool in a rotationally rigid manner.

17. Percussive tool according to one of the claims 14 to 16, wherein in the tool holder (25), in particular in the central body (30), a holding mechanism (50) for holding in place the tool engaged in the central body (30) through the orifice opening (36), in particular wherein a steel jacket (63) is arranged between the holding mechanism (50) and the central body (30), and in particular wherein the holding mechanism (50) has a holding body (52) that is movable transversely to the longitudinal axis against a spring force.

18. Percussive tool according to claim 17,
wherein the holding mechanism is arranged in the central body (30), and/or wherein the holding mechanism (50) is retained against the spring force by a pin (54) that is arranged in a blind hole (55) in the central body (30), wherein the blind hole (55) opens into a separation plane between the central body (30) and the front terminal part (31) such that the pin (54) is trapped in the blind hole (55) when the front terminal part (31) is mounted.

## Revendications

1. Outil de percussion, en particulier pour une utilisation chirurgicale, avec
une partie d'entraînement (3)
un organe d'entraînement (17) relié à la partie d'entraînement (3), et
un porte-outil (25) relié à l'organe d'entraînement (17) et agencé sur une face avant de la partie d'entraînement (3),
dans lequel la partie d'entraînement (3) est configurée de façon à exercer sur le porte-outil (25), via l'organe d'entraînement (17), des forces oscillatoires agissant dans une direction axiale (A),
**caractérisé en ce que** l'organe d'entraînement (17) est relié au porte-outil (25) via un accouplement (24), dans lequel l'accouplement (24) présente une première partie (80) s'amincissant vers l'extérieur et une deuxième partie (81) à cheval sur la première partie et s'élargissant vers l'extérieur, dans lequel, sous traction axiale, la deuxième partie est à cheval sur la première partie en étant écartée par la première partie,
**en ce qu'**entre le porte-outil (25) et l'organe d'entraînement (17) un organe de traction (85) est agencé pour relier les parties et générer la traction axiale, et
**en ce que** le porte-outil (25) présente une ouverture-embouchure et un siège agencé au niveau de l'ouverture-embouchure (36) pour recevoir un outil sans que ce dernier ne puisse tourner, dans lequel le siège présente une surface de réception (40) s'étendant perpendiculairement à la direction axiale (A), à partir de laquelle au moins quatre bossages (42) font saillie vers l'avant, symétriquement autour de l'ouverture-embouchure (36), par dessus la surface de réception (40) et entre lesquels il reste respectivement un emplacement pour recevoir des épaulements de l'outil.

2. Outil de percussion selon la revendication 1, dans lequel l'accouplement (24) est configuré en complémentarité de forme contre une rotation autour de la direction axiale (A), et en particulier dans lequel la première partie (80) présente un bossage en forme de selle.

3. Outil de percussion selon la revendication 1 ou 2, dans lequel l'organe de traction (85) présente une vis d'accouplement (85) s'étendant à travers l'accouplement (24), et en particulier dans lequel l'ouverture longitudinale (102) s'étend à travers la vis d'accouplement (85).

4. Outil de percussion selon la revendication 3, dans lequel la vis d'accouplement (85) est protégée par complémentarité de forme contre tout déplacement par au moins un corps d'arrêt, et en particulier dans lequel le corps d'arrêt est une goupille d'arrêt (87) qui est logée dans un trou de goupille (88) dans la partie terminale arrière (32) et est maintenue par au moins un verrou de goupille (90), et en particulier dans lequel le verrou de goupille (90) présente un corps de verrou agencé dans un trou de verrou (93) dans la partie terminale arrière (32), maintenu contre une force de ressort, dans lequel le corps de verrou peut être sorti du trou de goupille (88) contre la force de ressort.

5. Outil de percussion selon l'une quelconque des revendications 1 à 4, dans lequel l'accouplement (24) présente au moins une goupille d'accouplement (82) qui s'étend parallèlement à la direction axiale (A) à partir de l'organe d'entraînement (17) dans la partie terminale (32).

6. Outil de percussion selon l'une quelconque des revendications précédentes, dans lequel l'organe d'entraînement (17) s'étend d'un seul tenant à partir de l'accouplement (24) jusque dans la partie d'entraînement (3).

7. Outil de percussion selon l'une quelconque des revendications précédentes, dans lequel l'outil de percussion présente une ouverture longitudinale traversante, s'étendant dans la direction axiale (A) à travers le porte-outil (25) et la partie d'entraînement (3), à partir de la face avant de l'outil de percussion jusqu'à une face arrière de l'outil de percussion pour faire passer un composant en forme de barre.

8. Outil de percussion selon la revendication 7, dans lequel le porte-outil (25) présente une ouverture-embouchure (36) pour recevoir une extrémité d'outil, dans lequel l'ouverture longitudinale débouche sur l'ouverture-embouchure (36).

9. Outil de percussion selon la revendication 7 ou 8, dans lequel le porte-outil (25) est relié à la partie d'entraînement (3) via un organe d'entraînement (17), dans lequel l'ouverture longitudinale s'étend à travers l'organe d'entraînement (17).

10. Outil de percussion selon l'une quelconque des revendications 7 à 9, dans lequel la partie d'entraînement (3) présente un piston (4) entraîné dans un mouvement oscillatoire, l'ouverture longitudinale s'étendant à travers le piston (4).

11. Outil de percussion selon les revendications 9 et 10, dans lequel l'organe d'entraînement (17) présente un épaississement (16) agencé dans une cavité (15) à l'intérieur du piston (4), dans lequel l'ouverture longitudinale s'étend à travers la cavité (15) et une ouverture (104) dans le piston (4) s'étendant derrière la cavité, et en particulier dans lequel la cavité (15) est scellée par un joint d'étanchéité (20) par rapport à un espace de cylindre (6) dans lequel se trouve le piston (4).

12. Outil de percussion selon la revendication 11, dans lequel un tube (105) s'étend à partir du piston (4) vers l'arrière, dans lequel l'ouverture longitudinale s'étend à travers le tube (105), et en particulier dans lequel le tube (105) s'étend de manière déplaçable à travers un joint d'étanchéité (106) dans une paroi arrière (11) de l'outil de percussion.

13. Outil de percussion selon l'une quelconque des revendications précédentes, dans lequel des bossages (42) voisins présentent un espacement d'environ 10,05 mm.

14. Outil de percussion selon l'une quelconque des revendications précédentes, dans lequel le porte-outil (25) présente un corps central (30) en métal léger, une partie terminale avant (31) en acier et une partie terminale arrière (32) en acier.

15. Outil de percussion selon la revendication 14, dans lequel le corps central (30) consiste surtout en aluminium et/ou en magnésium et/ou en titane.

16. Outil de percussion selon l'une quelconque des revendications 14 ou 15, dans lequel la partie terminale avant (31) présente un siège pour recevoir un outil sans que ce dernier ne puisse tourner.

17. Outil de percussion selon l'une quelconque des revendications 14 à 16, dans lequel dans le porte-outil (25), en particulier dans le corps central (30), un mécanisme de maintien (50) est agencé pour maintenir l'outil mettant en prise le corps central (30) à travers une ouverture-embouchure (36), en particulier dans lequel une gaine d'acier (63) est agencée entre le mécanisme de maintien (50) et le corps central (30), et en particulier dans lequel le mécanisme de maintien (50) présente un corps de maintien (52) qui est déplaçable transversalement à l'axe longitudinal contre une force de ressort.

18. Outil de percussion selon la revendication 17, dans lequel le mécanisme de maintien est agencé dans le corps central (30), et/ou dans lequel le mécanisme de maintien (50) est protégé contre la force de ressort par une goupille (54) qui est agencée dans un trou borgne (55) dans le corps central (30), dans lequel le trou borgne (55) débouche sur une surface de séparation entre le corps central (30) et la partie terminale avant (31), de telle sorte que la goupille (54) est captive dans le trou borgne (55) une fois la partie terminale avant (31) installée.
